# EUROPEAN PATENT APPLICATION

(11) **EP 4 506 443 A1**
(43) Date of publication of application: **12.02.2025**
(21) Application number: 22934473.4
(22) Date of filing: 09.05.2022
(51) Int. Cl.: C12M 1/00

(54) **MICROFLUIDIC CHIP AND METHODS FOR CULTURING SINGLE CELL AND SCREENING AND EXPORTING CELL POPULATION**

(30) Priority: 31.03.2022 CN 202210356738
(71) Applicant: Shanghai Aurefluidics Technology Co., Ltd, Shanghai 201800 (CN)
(72) Inventor: WANG, Kun, Shanghai 201800 (CN); YANG, Miaomiao, Shanghai 201800 (CN); GUAN, Yimin, Shanghai 201800 (CN)
(74) Representative: Canzler & Bergmeier Patentanwälte Partnerschaft mbB
(86) International application number: PCT/CN2022/091720
(87) International publication number: WO 2023/184647

(57) **Abstract**

A microfluidic chip and methods for culturing a single cell and screening and exporting cell population. The chip (1) comprises a substrate layer, a flow channel layer, an inlet flow channel (2), an outlet flow channel (3), a plurality of common flow channels (4), and a plurality of functional units (5), wherein the flow channel layer is located below the substrate layer, and the materials of the flow channel layer comprise at least one of silicon dioxide, spin-on glass, non-photosensitive epoxy resin, and non-photosensitive polyimide; two ends of the common flow channel (4) are connected to the inlet flow channel (2) and the outlet flow channel (3), respectively; the functional unit (5) comprises a single cell introduction port (501), a cell culturing-screening chamber (502), a cell exporting chamber (503), a cell exporting port (504), and a driving element (505), the driving element (505) being used for providing power for liquid so as to introduce a single cell (6) entering the common flow channel (4) into the cell culturing-screening chamber (502) and export the cultured and screened target cell population from the cell exporting port (504). According to the microfluidic chip, the single cell culture, the cell population identification and screening, and the target cell population exporting are all completed on the chip, which simplifies experimental operations, reduces cross-contamination risk, and avoids fluorescence interference of the flow channel layer.

## Description

### TECHNICAL FIELD

The present disclosure belongs to the fields of microfluidics, development of cell lines, and screening of monoclonal antibodies., and relates to a microfluidic chip and a method for culturing single cells and screening and exporting cell populations.

### BACKGROUND

Cells are the fundamental units of life. Research at the single-cell level can uncover the intricate details of life processes and has broad applications in areas like monoclonal antibody screening and cell line culture. Single-cell isolation is crucial for this research. Currently, methods such as microneedle aspiration, limited dilution, microarray, and microfluidic sorting are used for single-cell isolation. However, these methods often face challenges like high operational difficulty, low efficiency, and the unintended isolation of multiple cells, which hinder subsequent culture and analysis. In monoclonal antibody screening and cell line culture, isolated single cells are typically cultured in well plates. After analyzing the titer and phenotype, well-performing cell groups are selected for large-scale culture. This process is labor-intensive, cumbersome, time-consuming, and inefficient. Therefore, in single-cell research, especially in monoclonal antibody screening and cell line development, there is an urgent need for an efficient method that integrates single-cell isolation, culture, cell population screening, and export, with simple operation.

### SUMMARY

In view of the above disadvantages of the related technology, the present disclosure provides a microfluidic chip and a single cell culture and cell population screening and exporting method, so as to solve the problems of large manpower occupation, tedious operation, long time consumption and low efficiency in the processes of isolation and culture of cells, screening of cell populations, or the like, in the related technology.

The presently disclosed integrated microfluidic chip includes:
a substrate layer;
a flow channel layer, located below the substrate layer, wherein a material of the flow channel layer includes at least one of silica, spin on glass, non-photosensitive epoxy resin, and non-photosensitive polyimide;
a liquid inlet flow channel and a liquid outlet flow channel, located in the flow channel layer and provided at an interval;
a plurality of common flow channels, located in the flow channel layer and provided at intervals, wherein two ends of each of the common flow channels are communicated with the liquid inlet flow channel and the liquid outlet flow channel, respectively; and
a plurality of functional units, wherein the functional units include single cell importing ports, cell culture screening chambers, cell exporting chambers, cell exporting ports, and driving elements, the single cell importing ports are located in the substrate layer and communicated with the common flow channels, the cell culture screening chambers, the cell exporting chambers and the cell exporting ports are all located in the flow channel layer, two ends of the cell culture screening chambers are communicated with the common flow channels and the cell exporting chambers, respectively, the cell exporting ports are located below the cell exporting chambers and communicated with the cell exporting chambers, the driving elements are located in the cell exporting chambers and face the cell exporting ports, and the driving elements are used for providing power for a liquid so as to draw single cells that are imported into the common flow channels through the single cell importing ports into the cell culture screening chambers and to export target cell populations that are cultured and screened in the cell culture screening chambers through the cell exporting ports.

Optionally, the liquid inlet flow channel, the liquid outlet flow channel, the common flow channels, the cell culture screening chambers and the cell exporting chambers all penetrate one surface of the flow channel layer that faces the substrate layer to expose the substrate layer, and the driving elements are bonded to a surface of the substrate layer.

Optionally, a material of the substrate layer includes silicon.

Optionally, an extension direction of the liquid inlet flow channel is parallel to an extension direction of the liquid outlet flow channel.

Optionally, an extension direction of the common flow channels is perpendicular to the extension direction of the liquid inlet flow channel.

Optionally, a thickness of the cell culture screening chambers is set to accommodate only a single layer of cells.

Optionally, on a plane where the flow channel layer is located and in a direction perpendicular to the cell culture screening chambers towards the cell exporting chambers, a width of the cell culture screening chambers is greater than a width of the cell exporting chambers.

Optionally, the single cell importing ports are used for receiving single cells ejected from single cell printing chips.

Optionally, the single cell printing chips include thermal bubble printing chips.

Optionally, the driving elements include one of heating films, piezoelectric nozzles, PDMS microvalves, solenoid valves, and peristaltic pumps.

Optionally, a quantity range of the functional units is 10-10,000.

The present disclosure further provides a method for culturing single cells and screening and exporting cell populations, including the following steps:
providing the microfluidic chip according to any one of the above embodiments, and injecting single cells into the common flow channels through the single cell importing ports;
after the cells settle naturally, using the driving elements to drive a liquid to flow, and drawing the single cells that are injected into the common flow channels into the cell culture screening chambers;
after the cells are cultured in the cell culture screening chambers for a preset time, importing a screening reagent into the cell culture screening chambers through a liquid inlet flow channel to identify and screen out target cell populations; and
transferring the target cell populations into a specified container through the cell exporting ports.

Optionally, before the single cells are injected into the common flow channels through the single cell importing ports, a cell culture solution is first perfused into the integrated microfluidic chip to expel air bubbles.

Optionally, after the single cells that are injected into the common flow channels are drawn into the cell culture screening chambers, the cell culture solution is perfused again to culture the cells.

Optionally, the cell culture solution is perfused by an injection pump.

Optionally, after the screening reagent is imported into the cell culture screening chambers, cell populations in the cell culture screening chambers are characterized by fluorescent images to screen out the target cell populations.

Optionally, the method for culturing single cells and screening and exporting cell populations is used for screening monoclonal antibody cell populations.

As mentioned above, the present disclosure provides a microfluidic chip for isolation and culture of single cells and screening and exporting of target cell populations. This chip enables single-cell culture, cell population identification and screening, and the export of target cell populations to be completed on-chip, increasing cell throughput, simplifying experimental operations, and reducing reagent usage and potential cross-contamination risks. Additionally, by incorporating thermal bubble printing technology, the entire process becomes more controllable, convenient, and efficient. Furthermore, in this microfluidic chip, the flow channel layer uses a silicon dioxide layer, spin-coated glass layer, non-photosensitive epoxy resin material layer, or non-photosensitive polyimide material layer to replace the traditional membrane, which can avoid the fluorescence interference of the flow channel layer itself.

### BRIEF DESCRIPTION OF DRAWINGS

FIG. 1 shows a plane layout diagram of flow channels in a microfluidic chip of the present disclosure.
FIG. 2 shows a longitudinal layout diagram of flow channels of functional units in the microfluidic chip of the present disclosure.
FIG. 3 shows a flow chart of a method for culturing single cells and screening and exporting cell populations of the present disclosure.

### Reference Numerals

- 1: Microfluidic chip
- 2: Liquid inlet flow channel
- 3: Liquid outlet flow channel
- 4: Common flow channel
- 5: Functional unit
- 501: Single cell importing port
- 502: Cell culture screening chamber
- 503: Cell exporting chamber
- 504: Cell exporting port
- 505: Driving element
- 6: Single cell
- 7: Single cell printing chip
- S 1-S4: Steps

### DETAILED DESCRIPTION OF EMBODIMENTS

Embodiments of the present disclosure are illustrated below with reference to particular specific examples. For those skilled in the art, other advantages and effects of the present disclosure can be easily understood according to the contents disclosed in the specification. The present disclosure may also be implemented or applied through other different specific embodiments, and various modifications or alterations of various details in the specification may also be made based on different viewpoints and applications without departing from the spirit of the present disclosure.

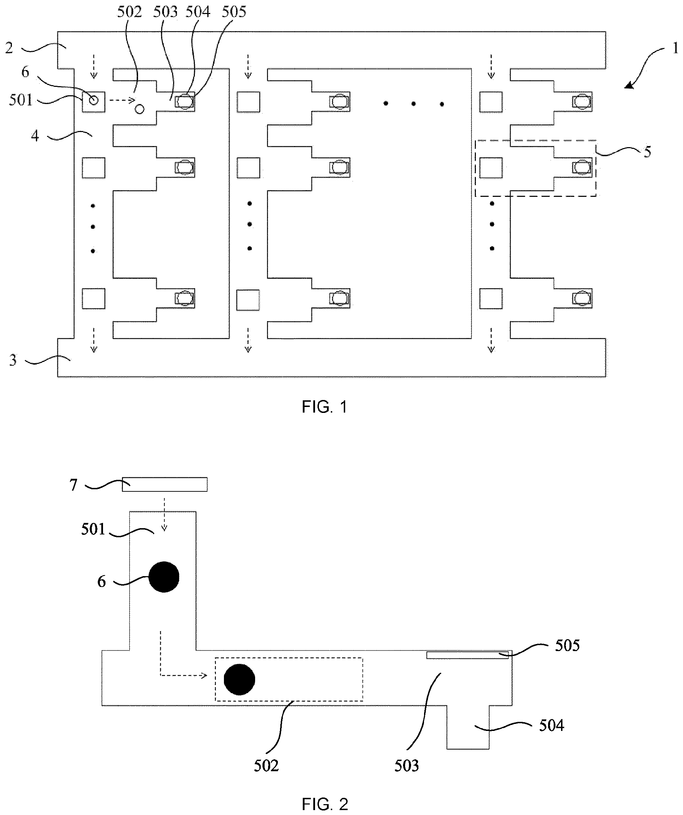

Referring to FIG. 1 to FIG. 3, it should be noted that schematic diagrams provided in the examples only illustrate the basic concept of the present disclosure in a schematic manner, such that the schematic diagrams only show components related to the present disclosure and are not drawn according to the number, shape and size of the components in actual implementation, the shape, quantity and proportion of various components in actual implementation can be arbitrarily changed, and the layouts shape of the components may also be more complex.

### Embodiment 1

The present embodiment provides a microfluidic chip, including a substrate layer, a flow channel layer, a liquid inlet flow channel, a liquid outlet flow channel, a plurality of common flow channels, and a plurality of functional units, wherein the flow channel layer is located below the substrate layer.

Specifically, a material of the flow channel layer includes at least one of silica, spin on glass (SOG, which is a type of spin-coated material made from polysiloxane), non-photosensitive epoxy resin, and non-photosensitive polyimide. Since a non-photosensitive material layer (such as a silica layer, a spin on glass layer, a non-photosensitive epoxy resin material layer, or a non-photosensitive polyimide material layer) is selected as the flow channel layer of the microfluidic chip of the present disclosure to replace a traditional dry film, the problem of fluorescence interference of the flow channel layer itself can be avoided.

For instance, a material of the substrate layer includes silicon, from which required circuit elements and circuits can be prepared. Since a silica layer, a spin on glass layer, a non-photosensitive epoxy resin material layer, or a non-photosensitive polyimide material layer is selected as the flow channel layer, not only can the problem of fluorescence interference of the flow channel layer itself be avoided, but also good bonding to a silicon substrate can be achieved, and the flow channel layer is easy to process on the silicon substrate by a micro-nano technology to form required flow channels. For example, a first flow channel layer can be formed first on the substrate layer, the first flow channel layer is subjected to graphical processing to obtain required flow channels, and then a second flow channel layer is formed on the first flow channel layer to seal the flow channels in the first flow channel layer.

For instance, referring to FIG. 1 and FIG. 2, FIG. 1 shows a plane layout diagram of flow channels in the microfluidic chip 1, and FIG. 2 shows a longitudinal layout diagram of flow channels of the functional units in the microfluidic chip.

Specifically, the liquid inlet flow channel 2 and the liquid outlet flow channel 3 are both located in the flow channel layer and provided at an interval. The plurality of common flow channels 4 is located in the flow channel layer and provided at intervals, and two ends of each of the common flow channels 4 are communicated with the liquid inlet flow channel 2 and the liquid outlet flow channel 3, respectively. The plurality of functional units 5 are arranged in an array. The functional units 5 include single cell importing ports 501, cell culture screening chambers 502, cell exporting chambers 503, cell exporting ports 504, and driving elements 505. The single cell importing ports 501 are located in the substrate layer and communicated with the common flow channels 4. The cell culture screening chambers 502, the cell exporting chambers 503 and the cell exporting ports 504 are all located in the flow channel layer. Two ends of each of the cell culture screening chambers 502 are communicated with the common flow channels 4 and the cell exporting chambers 503, respectively. The cell exporting ports 504 are located below the cell exporting chambers 503 and communicated with the cell exporting chambers 503. The driving elements 505 are located at bottoms of the cell exporting chambers 503 and face the cell exporting ports 504. The driving elements 505 are used for propelling liquid so as to draw single cells 6 that are imported into the common flow channels 4 through the single cell importing ports 501 into the cell culture screening chambers 502 and to export target cell populations that are cultured and screened in the cell culture screening chambers 502 through the cell exporting ports 504.

For instance, the liquid inlet flow channel 2, the liquid outlet flow channel 3, the common flow channels 4, the cell culture screening chambers 502 and the cell exporting chambers 503 all penetrate one surface of the flow channel layer that faces the substrate layer to expose the substrate layer, and the driving elements 505 are bonded to a surface of the substrate layer.

Specifically, the functional units 5 have the functions of isolation and culture of single cells, and screening and exporting of cell populations; thousands of functional units 5 can be integrated in the integrated microfluidic chip as needed to ensure high throughput of single cell culture and screening. For instance, a quantity range of the functional units 5 is 10-10,000, such as 1,000, 2,000, 5,000, etc.

For instance, a flow path of liquid is shown with dotted arrows in FIG. 1. An inlet of the liquid inlet flow channel 2 and an outlet of the liquid outlet flow channel 3 can be adjusted as required (e.g., can be located at one end of the corresponding flow channel or at a preset position in the middle of the flow channel), which are not shown in the figure. A culture solution and a reagent can be driven by external power (such as an injection pump) to facilitate cell culture and screening.

For instance, as shown in FIG. 1, an extension direction of the liquid inlet flow channel 2 is parallel to an extension direction of the liquid outlet flow channel 3, and an extension direction of the common flow channels 4 is perpendicular to the extension direction of the liquid inlet flow channel 2, such that the plurality of functional units 5 are arranged in a regular square array, cooperating with printing nozzles of single cell printing chips is facilitated, and cooperating with a cell receiving device is also facilitated. In other examples, the communication manner of the liquid inlet flow channel 2, the liquid outlet flow channel 3 and the common flow channels 4 can also be adjusted as required, which is not limited to the present example.

For instance, as shown in FIG. 2, a thickness of the cell culture screening chambers 502 is set to accommodate only a single layer of cells. For example, the thickness of the cell culture screening chambers 502 can be set to be match the size of the target cells, thereby ensuring that the cells are arranged in a single layer in the cell culture process and facilitating subsequent cell counting and fluorescence analysis.

For instance, as shown in FIG. 1, on a plane where the flow channel layer is located and in a direction perpendicular to the cell culture screening chambers 502 towards the cell exporting chambers 503, a width of the cell culture screening chambers 502 is greater than a width of the cell exporting chambers 503, which is conducive to making the screened cells sequentially enter the cell exporting chambers 503 and exported.

For instance, the driving elements 503 may include one of heating films, piezoelectric nozzles, PDMS (polydimethylsiloxane) microvalves, solenoid valves, and peristaltic pumps. In the present example, the driving elements 503 preferably adopt heating films, so as to form thermal bubble nozzles with the cell exporting chambers 503 and the cell exporting ports 504. The thermal bubble nozzles are prepared based on a micro-nano processing technology and integrated at a bottom of a microchannel. In the thermal bubble nozzles, liquid at an upper position is vaporized by instantaneous high temperature of the heating films, the liquid is then pushed by generated air bubbles to flow to be sprayed out of the nozzles, and then subsequent liquid is supplemented under the action of a capillary force, so as to provide power for continuous flow of liquid. The thermal bubble nozzles are controlled by an underlying circuit.

For instance, as shown in FIG. 2, the single cell importing ports 501 are used for receiving single cells 6 ejected from single cell printing chips 7, and positions of the single cell importing ports 501 are set such that after the single cells enter the common flow channels through the single cell importing ports 501, settling positions of the single cells are located in inlet regions of the cell culture screening chambers 502 to facilitate accurate entry into the cell culture screening chambers 502 under the driving action of the driving elements 505. The single cell printing chips 7 can adopt single cell printing chips disclosed by the Chinese Patent Application No. 108330065A or other suitable single cell printing chips to achieve isolation of the single cells and importing into the cell importing ports 501. Single cell printing chips including thermal bubble printing chips are preferably used in the present example, which cause less damage to cells and can efficiently and gently import the single cells to the single cell importing ports 501.

The integrated microfluidic chip of the present disclosure can be used for isolation and culture of single cells and screening and exporting of target cell populations, such that culture of single cells, identification and screening of cell populations and exporting of target cell populations are all completed on-chip, thereby increasing the cell throughput, simplifying experimental operation and reducing the reagent usage and the risk of cross contamination. Meanwhile, by incorporating the thermal bubble printing technology, the whole process can be more controllable, convenient and efficient.

### Embodiment 2

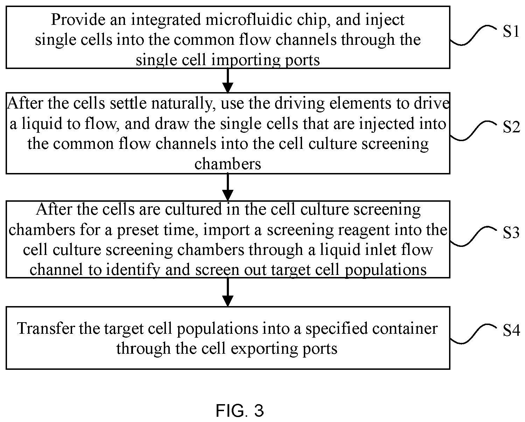

The present embodiment provides a method for culturing single cells and screening and exporting cell populations, referring to FIG. 3, showing a flow chart of the method, including the following steps:
S1: providing an integrated microfluidic chip in Embodiment 1, and injecting single cells into the common flow channels through the single cell importing ports;
S2: after the cells settle naturally, using the driving elements to drive liquid to flow, and drawing the single cells that are injected into the common flow channels into the cell culture screening chambers;
S3: after the cells are cultured in the cell culture screening chambers for a preset time, importing a screening reagent into the cell culture screening chambers through a liquid inlet flow channel to identify and screen out (that is, retain) target cell populations; and
S4: transferring the target cell populations into a specified container through the cell exporting ports.

For instance, in the step S1, before the single cells are injected into the common flow channels through the single cell importing ports, a cell culture solution is first perfused into the integrated microfluidic chip to expel air bubbles.

For instance, in the step S2, after the single cells that are injected into the common flow channels are drawn into the cell culture screening chambers, the cell culture solution is perfused into the integrated microfluidic chip again, such that nutrients enter the cell culture screening chambers by diffusion to culture the cells. In the present example, after all of the single cells enter the corresponding cell culture screening chambers, the cell culture solution is perfused using an injection pump.

For instance, in the step S3, after the screening reagent is imported into the cell culture screening chambers, cell populations in the cell culture screening chambers are characterized by fluorescent images to screen out the target cell populations. The screening reagent may include a phenotype identification antibody or other targets, depending on the target cells. In the present example, after the cells are cultured in the cell culture screening chambers for several days, the phenotype identification antibody or other targets are injected into the chip, and the target cell populations with good performance are screened out according to fluorescence characteristics of the cell populations.

For instance, in the step S4, a thermal bubble printing method is preferably used for transferring the target cell populations into the specified container for subsequent culture or analysis. The thermal bubble printing method has the advantages of small damage to cells, fast response, strong driving force, convenient control, easy integration and miniaturization, which guarantees convenient and efficient implementation of the whole process.

For instance, the method for culturing single cells and screening and exporting cell populations in the present example can be used for screening monoclonal antibody cell populations or other types of cell populations.

### Embodiment 3

The microfluidic chip in Embodiment 1 is used for screening monoclonal cell lines in the present embodiment. First, a cell culture solution is perfused into the chip to expel air bubbles. Then, perfusion of the culture solution is stopped, and transfected cells are placed in the form of single cells at the single cell importing cells of the chip through single cell printing chips. Thermal bubble nozzles are controlled to operate by an underlying circuit, so as to bring the single cells into the cell culture screening chambers. In order to obtain cell populations arranged as monolayer cells, a height of the cell culture screening chambers can be adjusted according to the size of the cells used. The culture solution is perfused again, and after the cells are cultured to obtain certain colonies, relevant fluorescent antibodies are injected through a reagent injection port to characterize the titer of antibodies secreted by each cell population. Then, target cell populations are screened out as required. In this case, the target cell populations are transferred into a specified container through the thermal bubble nozzles of the cell exporting ports again to complete screening of monoclonal antibody cell populations.

In summary, the present disclosure provides an integrated microfluidic chip for isolation and culture of single cells and screening and exporting of target cell populations. This chip enables single-cell culture, cell population identification and screening, and the export of target cell populations to be completed on-chip, increasing cell throughput, simplifying experimental operations, and reducing reagent usage and potential cross-contamination risks. Additionally, by incorporating thermal bubble printing technology, the entire process becomes more controllable, convenient, and efficient. Furthermore, in this microfluidic chip, the flow channel layer uses a silicon dioxide layer, spin-coated glass layer, non-photosensitive epoxy resin material layer, or non-photosensitive polyimide material layer to replace the traditional membrane, which can avoid the fluorescence interference of the flow channel layer itself. Thus, the present disclosure effectively overcomes various disadvantages of the prior art and has a high industrial utilization value.

The above examples are only illustrative description of the principles and effects of the present disclosure and are not used to limit the present disclosure. Any person familiar with the art can make modifications or alterations of the above examples without departing from the spirit and scope of the present disclosure. Therefore, all equivalent modifications or alterations that are made by persons with general knowledge skilled in the art without deviating from the spirit and technical concept revealed by the present disclosure shall still be covered by the claims of the present disclosure.

## Claims

1. A microfluidic chip, **characterized by** comprising:
a substrate layer;
a flow channel layer, located below the substrate layer, wherein a material of the flow channel layer comprises at least one of silica, spin on glass, non-photosensitive epoxy resin, and non-photosensitive polyimide;
a liquid inlet flow channel and a liquid outlet flow channel, located in the flow channel layer and provided at an interval;
a plurality of common flow channels, located in the flow channel layer and provided at intervals, wherein two ends of each of the common flow channels are communicated with the liquid inlet flow channel and the liquid outlet flow channel, respectively; and
a plurality of functional units, wherein the functional units comprise single cell importing ports, cell culture screening chambers, cell exporting chambers, cell exporting ports, and driving elements, the single cell importing ports are located in the substrate layer and communicated with the common flow channels, wherein the cell culture screening chambers, the cell exporting chambers and the cell exporting ports are all located in the flow channel layer, wherein two ends of each of the cell culture screening chambers are communicated with the common flow channels and the cell exporting chambers, respectively, wherein the cell exporting ports are located below the cell exporting chambers and communicated with the cell exporting chambers, the driving elements are located in the cell exporting chambers and face the cell exporting ports, and the driving elements propels liquid so as to draw single cells that are imported into the common flow channels through the single cell importing ports into the cell culture screening chambers and to export target cell populations that are cultured and screened in the cell culture screening chambers through the cell exporting ports.

2. The microfluidic chip according to claim 1, **characterized in that**: the liquid inlet flow channel, the liquid outlet flow channel, the common flow channels, the cell culture screening chambers and the cell exporting chambers all penetrate one surface of the flow channel layer that faces the substrate layer to expose the substrate layer, and the driving elements are bonded to a surface of the substrate layer.

3. The microfluidic chip according to claim 1, **characterized in that**: a material of the substrate layer comprises silicon.

4. The microfluidic chip according to claim 1, **characterized in that**: an extension direction of the liquid inlet flow channel is parallel to an extension direction of the liquid outlet flow channel.

5. The microfluidic chip according to claim 4, **characterized in that**: an extension direction of the common flow channels is perpendicular to the extension direction of the liquid inlet flow channel.

6. The microfluidic chip according to claim 1, **characterized in that**: a thickness of the cell culture screening chambers is set to accommodate only a single layer of cells.

7. The microfluidic chip according to claim 1, **characterized in that**: on a plane where the flow channel layer is located and in a direction perpendicular to the cell culture screening chambers towards the cell exporting chambers, a width of the cell culture screening chambers is greater than a width of the cell exporting chambers.

8. The microfluidic chip according to claim 1, **characterized in that**: the single cell importing ports are used for receiving single cells ejected from single cell printing chips.

9. The microfluidic chip according to claim 8, **characterized in that**: the single cell printing chips comprise thermal bubble printing chips.

10. The microfluidic chip according to claim 1, **characterized in that**: the driving elements comprise one of heating films, piezoelectric nozzles, PDMS microvalves, solenoid valves, and peristaltic pumps.

11. The microfluidic chip according to claim 1, **characterized in that**: a quantity range of the functional units is 10-10,000.

12. A method for culturing single cells and screening and exporting cell populations, **characterized by** comprising:
providing the microfluidic chip according to any one of claim 1 to claim 11, and injecting single cells into the common flow channels through the single cell importing ports;
after the cells settle naturally, using the driving elements to drive liquid to flow, and drawing the single cells that are injected into the common flow channels into the cell culture screening chambers;
after the cells are cultured in the cell culture screening chambers for a preset time, importing a screening reagent into the cell culture screening chambers through a liquid inlet flow channel to identify and screen out target cell populations; and
transferring the target cell populations into a specified container through the cell exporting ports.

13. The method for culturing single cells and screening and exporting cell populations according to claim 12, **characterized in that**: before the single cells are injected into the common flow channels through the single cell importing ports, a cell culture solution is first perfused into the integrated microfluidic chip to expel air bubbles.

14. The method for culturing single cells and screening and exporting cell populations according to claim 13, **characterized in that**: after the single cells that are injected into the common flow channels are drawn into the cell culture screening chambers, the cell culture solution is perfused again to culture the cells.

15. The method for culturing single cells and screening and exporting cell populations according to claim 14, **characterized in that**: the cell culture solution is perfused by an injection pump.

16. The method for culturing single cells and screening and exporting cell populations according to claim 12, **characterized in that**: after the screening reagent is imported into the cell culture screening chambers, cell populations in the cell culture screening chambers are **characterized by** fluorescent images to screen out the target cell populations.

17. The method for culturing single cells and screening and exporting cell populations according to claim 12, **characterized in that**: the method for culturing single cells and screening and exporting cell populations is used for screening monoclonal antibody cell populations.
